# EUROPEAN PATENT APPLICATION

(11) **EP 3 939 629 A1**
(43) Date of publication of application: **19.01.2022**
(21) Application number: 19919483.8
(22) Date of filing: 12.12.2019
(51) Int. Cl.: A61L 29/06

(54) **MEDICAL TUBE AND PREPARATION METHOD THEREFOR**

(30) Priority: 11.03.2019 CN 201910181383
(71) Applicant: Accupath Medical (Jiaxing) Co., Ltd., Jiaxing, Zhejiang 314006 (CN)
(72) Inventor: QIN, Minglin, Jiaxing, Zhejiang 314006 (CN); LI, Zhaomin, Jiaxing, Zhejiang 314006 (CN); LIU, Yifan, Jiaxing, Zhejiang 314006 (CN); ZHANG, Xinhua, Jiaxing, Zhejiang 314006 (CN); LI, Ruipei, Jiaxing, Zhejiang 314006 (CN); DENG, Zhihua, Jiaxing, Zhejiang 314006 (CN); QUE, Yiyun, Jiaxing, Zhejiang 314006 (CN)
(74) Representative: Dauncey, Mark Peter
(86) International application number: PCT/CN2019/124944
(87) International publication number: WO 2020/181857

(57) **Abstract**

Disclosed are a medical tube and a preparation method therefor. The preparation method for the medical tube comprises the following steps: S1: subjecting levodopa to a self-polymerization reaction to obtain a poly-levodopa solution; S2: soaking a polyether block amide tube in a cationic polyelectrolyte to introduce positive ions onto the surface of the polyether block amide tube; S3: soaking the treated polyether block amide tube in step S2 in the poly-levodopa solution prepared in step S1, such that a poly-levodopa coating is formed and adhered to the surface of the polyether block amide tube; and S4: placing the polyether block amide tube having the poly-levodopa coating into a modified material solution for reaction, so as to obtain a polyether block amide tube with a modified coating. By using levodopa to form a poly-levodopa coating on the surface of the polyether block amide tube, and then modifying the coating by means of a grafting method, the biocompatibility, hydrophilcity and interface adhesion properties of the medical tube are improved.

## Description

### TECHNICAL FIELD

The present application relates to the technical field of medical devices, in particular to a medical tube and a method for preparing the same.

### BACKGROUND

Polyether block amide (Pebax), one of thermoplastic elastomers (TPE), is a type of thermoplastic multi-block copolymer composed of polyamide (PA) as a hard segment and polyether (PE) as a soft segment. The polyether block amide (Pebax) has good biodegradability, shape memory property and biocompatibility, and has been widely used in the field of biomedicine. However, there are still several deficiencies in the biological properties of the polyether block amide (Pebax) and its bonding performance with other materials. Therefore, it is necessary to modify the surface of the polyether block amide (Pebax) tube to improve its biocompatibility, hydrophilicity and its bonding performance with other materials.

### SUMMARY

Conventional treatment methods such as plasma, chemical grafting, and chemical etching have been used to introduce active groups on the surface of the tubes to enhance the interface bonding performance between the tube and other substrates, however, they have various disadvantages, such as damage to the tube body which causes declined mechanical properties, harsh reaction conditions, high requirements for equipment, and easily causing pollution. Therefore, it is of great significance to seek a new method for preparing tubes.

### TECHNICAL SOLUTIONS

The technical problem to be solved by the present disclosure is to provide a medical tube and a method for preparing the same, in order to improve the biocompatibility, hydrophilicity and interface bonding performance of the surface of the tube.

In order to solve the above technical problems, the present disclosure provides a method for preparing a medical tube, which includes steps of: S1, performing a self-polymerization reaction of levodopa to obtain a polylevodopa solution; S2, soaking a polyether block amide tube in a solution of cationic polyelectrolyte to introduce positive ions on a surface of the polyether block amide tube; S3, soaking the polyether block amide tube treated in step S2 in the polylevodopa solution prepared in step S1 to form a polylevodopa coating on the surface of the polyether block amide tube; and S4, placing the polyether block amide tube with the polylevodopa coating in a solution of modified material for reaction to obtain a polyether block amide tube modified by the coating.

Preferably, step S1 includes: dissolving the levodopa in water to obtain a levodopa solution, adjusting the solution to pH 8.0-9.0, and performing the self-polymerization reaction to obtain the polylevodopa solution.

Preferably, at least one of sodium hydroxide, tris(hydroxymethyl)aminomethane and sodium carbonate compound is added to the levodopa solution to adjust the solution to pH 8.0 to 9.0.

Preferably, the self-polymerization reaction in step S1 is performed for 16 hours to 24 hours.

Preferably, the cationic polyelectrolyte in step S2 is in a concentration of 1.0 mg/mL to 3.0 mg/mL.

Preferably, the solution of cationic polyelectrolyte in step S2 contains sodium chloride. The sodium chloride is contained in a concentration of 1.0 mg/mL to 3.0 mg/mL.

Preferably, the solution of cationic polyelectrolyte in step S2 is a solution of poly(diallyldimethylammonium chloride) or a solution of sodium polyacrylate.

Preferably, the soaking in steps S2 and S3 is performed for 5 min to 20 min, and the soaking is followed by rinsing with water and drying with nitrogen.

Preferably, steps S2 and S3 are repeated 1 to 8 times.

Preferably, the modified material in step S4 is at least one selected from the group consisting of heparin, polyethylene glycol, phosphorylcholine, glycidyl methacrylate, hydroxyethyl methacrylate, phosphorylcholine and albumin.

Preferably, step S4 includes: soaking the polyether block amide tube with the polylevodopa coating in the solution of modified material, adjusting the solution to pH 8.5 to 9.0, and performing the reaction for 8 hours to 24 hours, followed by rinsing with water, to obtain the polyether block amide tube modified by the coating.

Preferably, the modified material is heparin. A solution of the heparin is in a concentration of 20 mg/mL to 40 mg/mL.

Preferably, in step S4, the polyether block amide tubes with the polylevodopa coating are placed in solutions of modified material in different concentrations, so that the polylevodopa coatings are coated with modified material coatings of different thicknesses on an outside thereof, respectively.

In order to solve the above technical problems, the present disclosure also provides a medical tube manufactured by the above preparation method.

### BENEFICIAL EFFECT

Compared with the prior art, the present disclosure has the following beneficial effects: for the medical tube and the method for preparing the same provided in the present disclosure, the self-polymerization of the levodopa is performed to introduce active functional groups such as hydroxyl and amino groups on the surface of the polyether block amide (Pebax) tube. Then, the active functional groups are reacted with the modified materials such as heparin, polyethylene glycol (PEG), phosphocholine, glycidyl methacrylate (GMA), hydroxyethyl methacrylate (HEMA), or albumin. As a result, the biocompatibility, hydrophilicity and interfacial bonding performance of the polyether block amide (Pebax) tube are improved, large-scale batch production, simplified production process, and increased production capacity can be achieved, and the requirements of different products can be met.

### DETAILED DESCRIPTION OF THE EMBODIMENTS

The present disclosure will be further described below in conjunction with examples.

In the present disclosure, levodopa, a secretion of mussels, is used to form a coating on a surface of a polyether block amide (Pebax) tube, and then the coating is modified by a grafting method. Thereby, the biocompatibility, hydrophilicity and interface bonding performance of the precision tube for interventional medical devices is improved.

Mussels, a kind of crustaceans that are ubiquitous in coastal waters, especially in cold waters, can secrete super-adhesive proteins to firmly adhere themselves to a surface of any material such as metal, glass, polymer, and mineral. This adhesive protein can quickly be solidified in a humid environment and strongly interact with the matrix material. Levodopa is the key to the adhesion behavior of the adhesive protein of the mussel.

The method for preparing the medical tube provided in the present disclosure, which uses the levodopa to improve the biocompatibility, hydrophilicity and interface bonding performance of the medical tube, includes the following steps.

### Step S1. Self-polymerization reaction of levodopa

A predetermined amount of levodopa is dissolved in a predetermined amount of water to obtain a levodopa solution. The solution is adjusted to pH 8.0 to 9.0 using a base, and a self-polymerization reaction is performed at room temperature for 16 hours to 24 hours to obtain a polylevodopa solution. The base may be at least one of sodium hydroxide, tris(hydroxymethyl)aminomethane (Tris) and sodium carbonate and the like.

### Step S2. Introduction of positive ions on a surface of the tube

A rinsed polyether block amide (Pebax) tube is soaked in a solution of cationic polyelectrolyte for 5 min to 20 min, and then rinsed with water and dried with nitrogen. The solution of cationic polyelectrolyte may be a solution of poly(diallyldimethylammonium chloride) (PDDA) or a solution of sodium polyacrylate. The solution of cationic polyelectrolyte may contain sodium chloride. The sodium chloride is contained in a concentration of 1.0 mg/mL to 3.0 mg/mL.

### Step S3. Attachment of polylevodopa coating on the surface of the tube

The tube processed in step S2 is soaked in the polylevodopa solution prepared in step S1 for 5 min to 20 min, and then rinsed with water and dried with nitrogen.

In this step, polylevodopa can be effectively bound to the surface of the tube, since polylevodopa has negative carboxylic acid ions which can be bonded with the positive ions on the surface of the tube. Moreover, due to electrostatic adsorption, the polylevodopa is adsorbed on the surface of the tube to form a dense coating, thereby introducing a large number of active functional groups, such as hydroxyl, carboxyl, and amino groups, on the surface of the tube.

### Step S4. Modification of polylevodopa coating on a surface of the tube

The polyether block amide (Pebax) tube with the polylevodopa coating is placed in a prepared solution of modified material, and then the solution is adjusted to pH 8.5 to 9.0 using phosphate buffer. The modified material is at least one selected from the group consisting of heparin, polyethylene glycol (PEG), phosphorylcholine, glycidyl methacrylate (GMA), hydroxyethyl methacrylate (HEMA), phosphorylcholine and albumin. The modified material may be the heparin. A solution of the heparin is in a concentration of 20 mg/mL to 40 mg/mL. The active functional groups on the polylevodopa coating are easily reacted with the functional groups carried by the above-mentioned modified material, so that the polylevodopa coating is coated with a layer of modified material coating on its outside, which improves the biological properties of the tube. After reacted for 8 hours to 24 hours at room temperature, it is rinsed with distilled water and dried to obtain a polyether block amide (Pebax) tube modified by the coating. In addition, since the thickness of the modified material coating on the polylevodopa coating is related to the concentration of the modified material solution, the polyether block amide tubes with the polylevodopa coating can be placed in solutions of modified material in different concentrations to obtain polyether block amide tubes with modified material coatings of different thicknesses, which can meet the requirements of different products.

In addition, the above steps S2 and S3 can be repeated, and different number of repetitions may result in the polyether block amide (Pebax) tubes with different thicknesses of polylevodopa coating on the surface. Steps S2 and S3 are repeated preferably 1 to 8 times.

In Example 1, a Pebax medical tube was cut to have a length of 15 cm. A surface of the tube was rinsed with 75% ethanol and deionized water to remove impurities, and then placed in a vacuum oven with temperature set to 80°C to a constant weight. Levodopa was dissolved in water to obtain a levodopa solution, and the solution was adjusted to pH 8.0 to 9.0 using a base. A self-polymerization reaction was performed at room temperature for 16 hours to 24 hours to obtain a polylevodopa solution. The base may be sodium hydroxide, tris(hydroxymethyl)aminomethane (Tris), sodium carbonate or the like. The rinsed tube was soaked in a solution of poly(diallyldimethylammonium chloride) (PDDA) in concentration of 1.0 mg/mL for 5 min, and then rinsed with water and dried with nitrogen. Then, the tube was soaked in the polylevodopa solution (pH 8.0-9.0, conc.=1.0 to 3.0 g/L(w/v)) for 5 min and rinsed again with water and dried in vacuum. The above operation was repeated (1 to 8 times) to obtain a polyether block amide tube with different thicknesses of polylevodopa coating. Then, the polyether block amide tube was soaked in a prepared solution of heparin in concentration of 20 mg/mL (adjusted to pH 9.0 using phosphate buffer), reacted for 8 hours to 24 hours at room temperature, and rinsed with water, obtaining a modified polyether block amide tube.

### Example 2

The product in this example has the same structure and the same producing process as example 1 except for the concentration of heparin, which is 30 mg/mL in this example.

### Example 3

The product in this example has the same structure and the same producing process as example 1 except for the concentration of heparin, which is 40 mg/mL in this example.

The preparation method provided in the present disclosure can ensure the precision of the tube, so that large-scale batch production can be achieved, and the requirements of different products can be met. Also, the biocompatibility of the tube can be improved, and

| Polyether block amide (Pebax 3533) | Concentration of heparin (mg/mL) | Hemolysis rate (%) | Cell proliferation rate (%) |
|---|---|---|---|
| 1# | 0 | 0.3 | 79 |
| 2# | 20 | 0.1 | 85 |
| 3# | 30 | 0 | 92 |
| 4# | 40 | 0 | 100 |

simplified production process and increased production capacity can be achieved. The medical tube can be a thermoplastic elastomer of a lower-hard segment, such as polyether block amide (Pebax) 3533 tube, with its biocompatibility significantly improved. The biological properties thereof are shown in Table 1.

### Table 1. Biological properties of the polyether block amide (Pebax 3533) tubes in different heparin concentrations

In the table, the hemolysis rate refers to a percentage of the tube that dissolves into the blood in the entire tube after the tube enters a human body. The cell proliferation rate refers to a percentage of cells newly produced by the human body in original human cells after the tube enters the human body. After the tube enters the human body, the hemolysis rate should not be too high, preferably in a range from 0 to 0.1%. The cell proliferation rate must be 85% or more, so that the tube can be used as a medical tube. It can be seen from Table 1 that the polyether block amide tube soaked in the solution of modified material, which is the heparin, can have greatly reduced hemolysis rate and increased cell proliferation rate, thereby obtaining significantly improved biocompatibility and meeting the requirements of medical tubes.

In summary, the medical tube and the method for preparing the same provided in the present disclosure have the following advantages.
(1) The surface of the tube is coated with the polylevodopa coating by a method of coating the polylevodopa layer by layer. This coating has a large number of active functional groups, and is thus easily reacted with the functional groups carried by the modified material, so that the polylevodopa coating is coated with the modified material coating on its outside, which improves the biological properties of the tube.
(2) The process is simple, environmentally friendly, and pollution-free, and continuous large-scale production can be carried out.
(3) The thickness of the coating is controllable, which is beneficial to regulating the bonding force between the coating and the tube. In addition, the medical polymer tube can not only have the characteristics of high toughness and high flexibility, but also have biocompatibility and lubricity, which can also meet the clinical requirements of precision tube for minimally invasive interventional medical devices.

Although the present disclosure has been disclosed as above in preferred embodiments, they are not intended to limit the present disclosure. Some modifications and improvements can be made by any one skilled in the art without departing from the spirit and scope of the present disclosure. Thus, the protection scope of the present disclosure should be defined by the claims.

## Claims

1. A method for preparing a medical tube, comprising steps of:
S1, performing a self-polymerization reaction of levodopa to obtain a polylevodopa solution;
S2, soaking a polyether block amide tube in a solution of cationic polyelectrolyte to introduce positive ions on a surface of the polyether block amide tube;
S3, soaking the polyether block amide tube treated in the step S2 in the polylevodopa solution prepared in the step S1 to form a polylevodopa coating on the surface of the polyether block amide tube; and
S4, placing the polyether block amide tube with the polylevodopa coating in a solution of modified material for reaction, to obtain a polyether block amide tube modified by the coating.

2. The method for preparing a medical tube according to claim 1, wherein the step S1 comprises: dissolving the levodopa in water to obtain a levodopa solution, adjusting the solution to pH 8.0-9.0, and performing the self-polymerization reaction to obtain the polylevodopa solution.

3. The method for preparing a medical tube according to claim 2, wherein at least one of sodium hydroxide, tris(hydroxymethyl)aminomethane and sodium carbonate compound is added to the levodopa solution to adjust the solution to pH 8.0 to 9.0.

4. The method for preparing a medical tube according to claim 2, wherein the self-polymerization reaction in the step S1 is performed for 16 hours to 24 hours.

5. The method for preparing a medical tube according to claim 1, wherein the solution of cationic polyelectrolyte in the step S2 is in a concentration of 1.0 mg/mL to 3.0 mg/mL.

6. The method for preparing a medical tube according to claim 1, wherein the solution of cationic polyelectrolyte in the step S2 contains sodium chloride, and the sodium chloride is contained in a concentration of 1.0 mg/mL to 3.0 mg/mL.

7. The method for preparing a medical tube according to claim 1, 5, or 6, wherein the solution of cationic polyelectrolyte in the step S2 is a solution of poly diallyldimethylammonium chloride or a solution of sodium polyacrylate.

8. The method for preparing a medical tube according to claim 1, wherein the soaking in the steps S2 and S3 is performed for 5 min to 20 min, and the soaking is followed by rinsing with water and drying with nitrogen.

9. The method for preparing a medical tube according to claim 1, wherein the steps S2 and S3 are repeated 1 to 8 times.

10. The method for preparing a medical tube according to claim 1, wherein the modified material in the step S4 is at least one selected from the group consisting of heparin, polyethylene glycol, phosphorylcholine, glycidyl methacrylate, hydroxyethyl methacrylate, phosphorylcholine and albumin.

11. The method for preparing a medical tube according to claim 10, wherein the step S4 comprises: soaking the polyether block amide tube with the polylevodopa coating in the solution of modified material, adjusting the solution to pH 8.5 to 9.0, and performing the reaction for 8 hours to 24 hours, followed by rinsing with water, to obtain the polyether block amide tube modified by the coating.

12. The method for preparing a medical tube according to claim 10, wherein the modified material is heparin, and a solution of the heparin is in a concentration of 20 mg/mL to 40 mg/mL.

13. The method for preparing a medical tube according to claim 1, wherein in the step S4, the polyether block amide tubes with the polylevodopa coating are placed in solutions of modified material in different concentrations, so that the polylevodopa coatings are coated with modified material coatings of different thicknesses on an outside thereof, respectively.

14. A medical tube, manufactured by the method for preparing a medical tube according to any one of claims 1 to 13.
